# EUROPEAN PATENT SPECIFICATION

(11) **EP 4 050 097 B1**
(45) Date of publication and mention of the grant of the patent: **25.09.2024**
(21) Application number: 22157001.3
(22) Date of filing: 16.02.2022
(51) Int. Cl.: C12N 5/00, C12N 5/071, A01N 1/02

(54) **METHOD OF PRODUCING FROZEN RENAL CELLS AND FROZEN RENAL CELL**
VERFAHREN ZUR HERSTELLUNG VON GEFRORENEN NIERENZELLEN UND GEFRORENE NIERENZELLE
PROCÉDÉ DE PRODUCTION DE CELLULES RÉNALES CONGELÉES ET CELLULE RÉNALE CONGELÉE

(30) Priority: 26.02.2021 JP 2021031068
(43) Date of publication of application: 31.08.2022
(73) Proprietor: Nikkiso Co., Ltd., Tokyo 150-6022 (JP)
(72) Inventor: Takahashi, Etsushi, Kanazawa-shi, Ishikawa, 920-0177 (JP)
(74) Representative: Hoffmann Eitle

(56) References cited:
- WO-A1-2020/223125
- US-A1- 2020 291 361
- KUMAR SANTHOSH V. ET AL: "Kidney micro-organoids in suspension culture as a scalable source of human pluripotent stem cell-derived kidney cells", DEVELOPMENT, vol. 146, no. 5, 1 March 2019 (2019-03-01), GB, XP055928514, ISSN: 0950-1991, Retrieved from the Internet <URL:https://journals.biologists.com/Toolbox/DownloadCombinedArticleAndSupplmentPdf?resourceId=49020&multimediaId=1858745&pdfUrl=/cob/content_public/journal/dev/146/5/10.1242_dev.172361/7/dev172361.pdf> DOI: 10.1242/dev.172361
- GIJZEN LINDA ET AL: "Culture and analysis of kidney tubuloids and perfused tubuloid cells-on-a-chip", NATURE PROTOCOLS, NATURE PUBLISHING GROUP, GB, vol. 16, no. 4, 5 March 2021 (2021-03-05), pages 2023 - 2050, XP037424463, ISSN: 1754-2189, [retrieved on 20210305], DOI: 10.1038/S41596-020-00479-W

## Description

### BACKGROUND

### Technical Field

The present invention relates to a method of producing a renal cell culture as claimed.

### Related Art

Drugs administered to the living body are absorbed into the living body, and then excreted from the blood into the urine in the proximal tubule in the kidney. Thus, renal damage is often caused by the nephrotoxicity of the drugs. In drug discovery research, it is very important to examine the pharmacokinetics in the kidney in order to determine the action of the drugs.

Therefore, as a drug discovery support device, there is a demand for development of an assay system capable of evaluating pharmacokinetics and toxicity using renal cells having normal physiological functions. In recent years, many researchers have developed renal cells derived from human iPS cells, a perfusion culture system, and the like. Since proximal tubular epithelial cells are most susceptible to drugs, there is a particular need for proximal tubular epithelial cells available for research.

It is known that when renal cells are cultured in a flat bottom culture plate different from the in vivo environment, the cultured renal cells lose many of the functions originally possessed by the kidney. Conventionally, cells collected from human kidneys have been commonly two-dimensionally plane-cultured on a flat bottom culture plate in a state of being dispersed by enzyme treatment. Cultured proximal tubular epithelial cells have not been utilized in drug discovery research because the proximal tubular epithelial cells have shown less normal renal pharmacokinetic and toxic responses due to the dedifferentiation phenomenon.

It has been found that the renal functions are greatly improved by causing the proximal tubular epithelial cells with lost original physiological functions of the kidney to form aggregates and culturing the resultant aggregates for a certain period (WO 2018/186185 A). In order to use the aggregates of proximal tubular epithelial cells with improved renal functions for drug discovery research, it is necessary to produce a large amount of the aggregates and preserve a certain amount of the aggregates.

In order to preserve the cells in a stable state, cryopreservation is the best means. In a method of freezing cells that has been conventionally and commonly performed, first, cell-cell adhesion is loosened with a digestive enzyme (trypsin or the like), and cells are dispersed one by one. Subsequently, the suspension containing the dispersed cells is centrifuged, a liquid containing a cryoprotectant is added to the collected cells, and the resultant mixture is frozen (JP H06-46840 A).

However, when the conventional freezing method is applied in the case of freezing aggregates of proximal tubular epithelial cells, even if attempts are made to produce aggregates by the same method after thawing, the aggregates remain in a dispersed state. Thus, it is difficult to form aggregates. Further, the cells frozen by such a method have some problems that the functions of the kidney cannot be maintained because the gene expression responsible for the function of the kidney is reduced after thawing, and the cell viability after culture is low.

### Citation List

### Patent Literature

Patent Literature 1: WO 2018/186185 A
Patent Literature 2: JP H06-46840 A

### SUMMARY

### Technical Problem

An object of the present invention is to provide frozen renal cells which have high cell viability after thawing and in which physiological functions of the kidney are maintained, and a renal cell culture in which the physiological functions of the kidney are maintained.

### Solution to Problem

One embodiment according to the present invention is a method of producing a frozen-state renal cell culture as claimed, including: a recovery step of recovering cultured renal cells; and a freezing step of freezing the renal cells recovered in the recovery step, in which the renal cells are recovered as an aggregate in the recovery step, and the aggregate is frozen while an aggregated state of the aggregate is substantially maintained in the freezing step.

Disclosed are furthermore frozen-state renal cells produced by a method including: a recovery step of recovering cultured renal cells; and a freezing step of freezing the renal cells recovered in the recovery step, in which the renal cells are recovered as an aggregate in the recovery step, and the aggregate is frozen while an aggregated state of the aggregate is substantially maintained in the freezing step.

### Advantageous Effects of Invention

The present invention allows to obtain a culture of frozen renal cells which have high cell viability after thawing and in which physiological functions of the kidney are maintained, and a renal cell culture in which the physiological functions of the kidney are maintained.

### BRIEF DESCRIPTION OF DRAWINGS

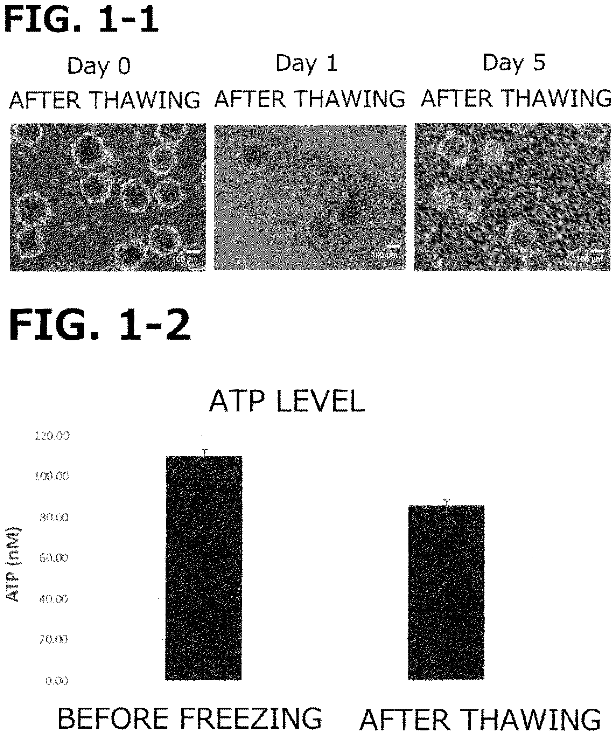

FIG. 1-1 is a view of optical microscope photographs each showing the form of aggregates obtained after thawing of proximal tubular epithelial cells frozen in an aggregated state;
FIG. 1-2 is a diagram illustrating ATP levels (cell viability) in proximal tubular epithelial cells frozen in an aggregated state;
FIG. 2-1 is a diagram illustrating, in comparison with human renal cortex, results of OAT1 gene expression analysis by a real-time PCR method of proximal tubular epithelial cells frozen in an aggregated state;
FIG. 2-2 is a diagram illustrating, in comparison with human renal cortex, results of OCT2 gene expression analysis by a real-time PCR method of proximal tubular epithelial cells frozen in an aggregated state;

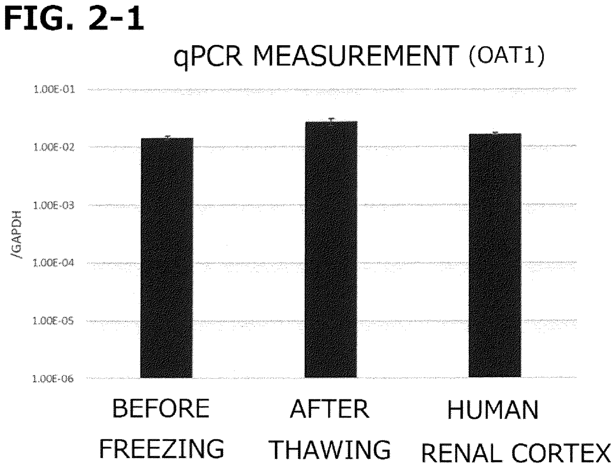

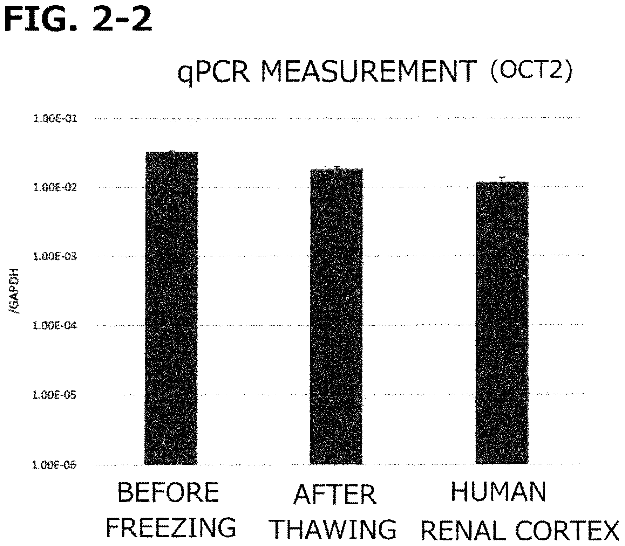

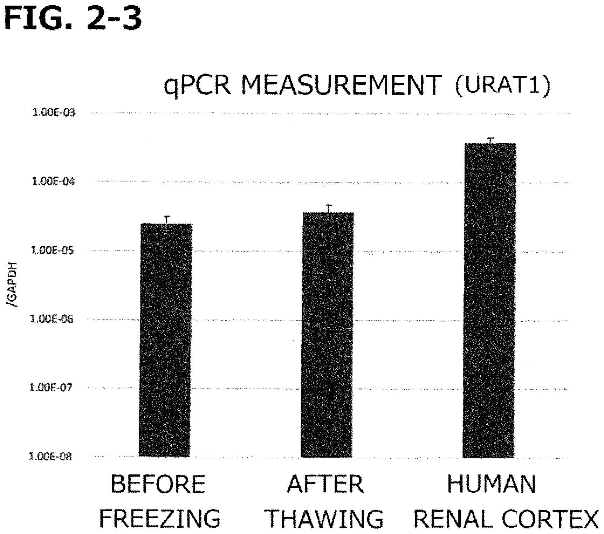

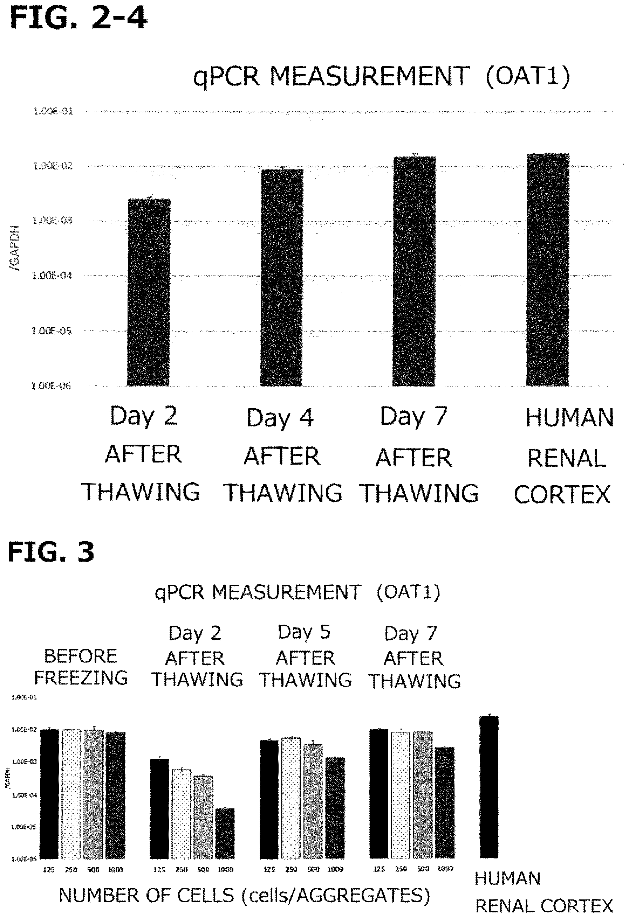

FIG. 2-3 is a diagram illustrating, in comparison with human renal cortex, results of URAT1 gene expression analysis by a real-time PCR method of proximal tubular epithelial cells frozen in an aggregated state;
FIG. 2-4 is a diagram illustrating, in comparison with human renal cortex, results of OAT1 gene expression analysis by a real-time PCR method of proximal tubular epithelial cell aggregates obtained by thawing and shake-culturing aggregates in a suspended state;
FIG. 3 is a diagram illustrating, in comparison with human renal cortex, results of OAT1 gene expression analysis by a real-time PCR method of proximal tubular epithelial cell aggregates frozen in different cell numbers;

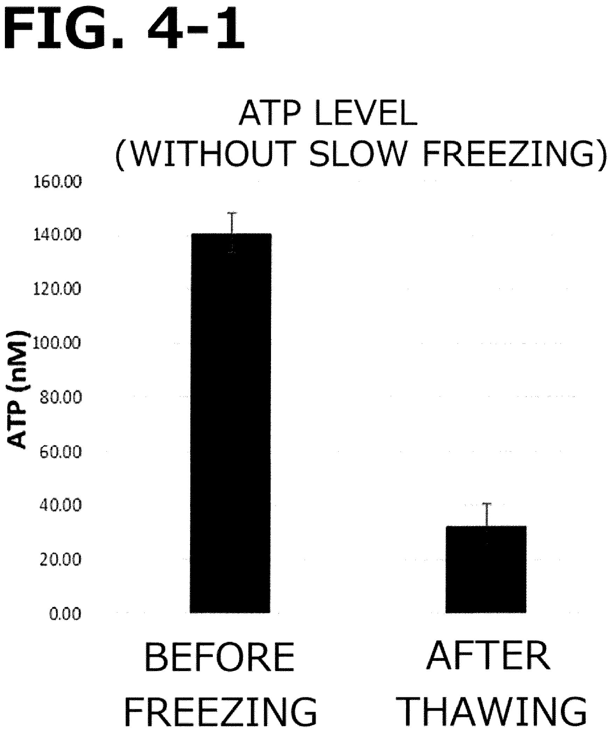

FIG. 4-1 is a diagram illustrating ATP levels (cell viability) of proximal tubular epithelial cell aggregates in the case of changing the freezing method;

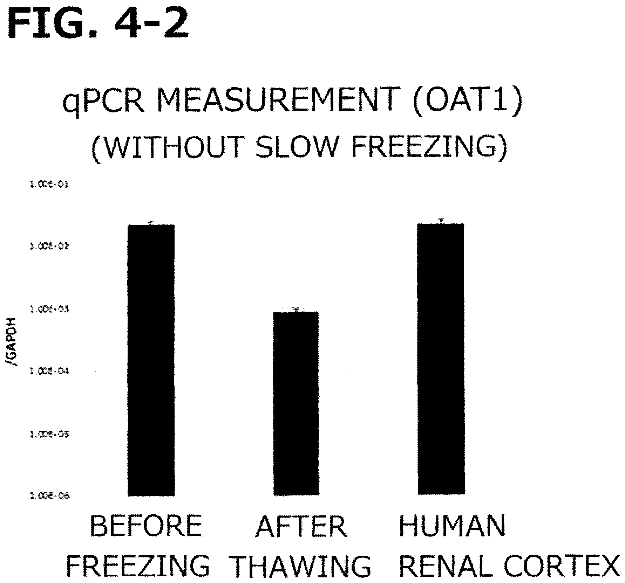

FIG. 4-2 is a diagram illustrating, in comparison with human renal cortex, results of OAT1 gene expression analysis by a real-time PCR method of proximal tubular epithelial cell aggregates in the case of changing the freezing method;

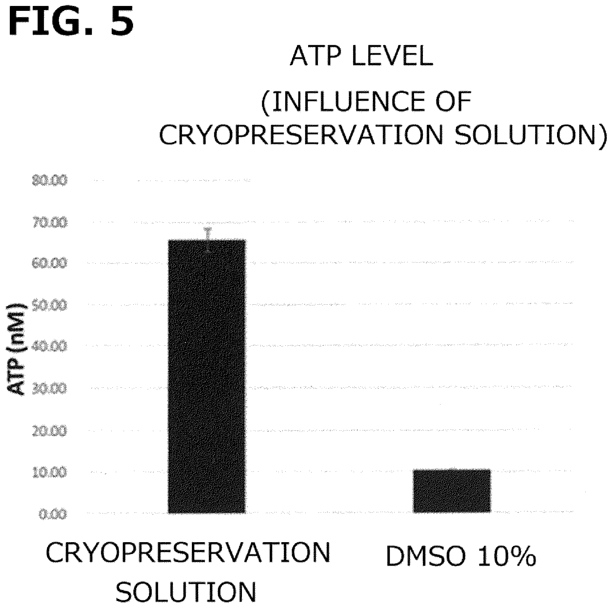

FIG. 5 is a diagram illustrating ATP levels (cell viability) of proximal tubular epithelial cell aggregates after thawing in the case of adding different cryopreservation solutions at the time of freezing;

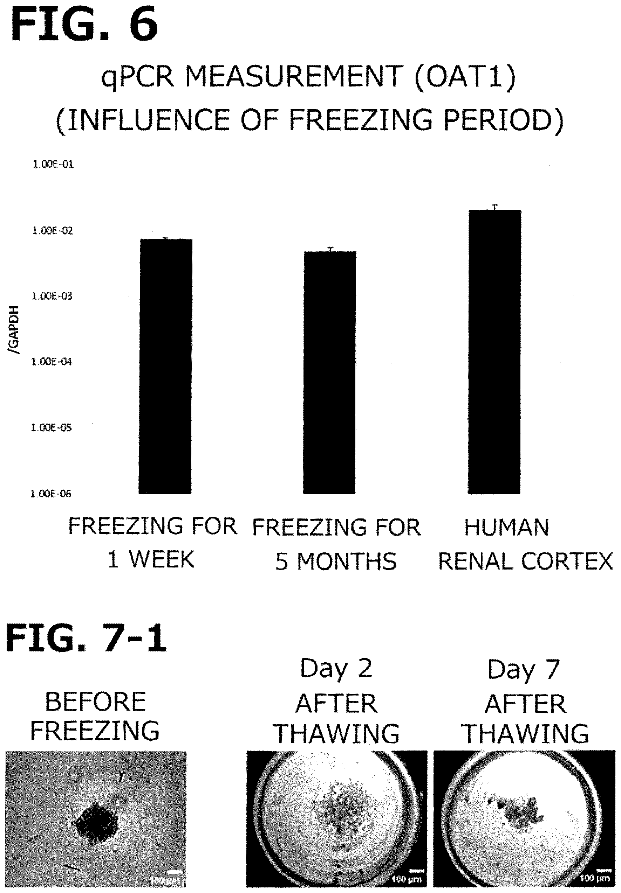

FIG. 6 is a diagram illustrating results of OAT1 gene expression analysis by a real-time PCR method of proximal tubular epithelial cell aggregates after thawing in the case of changing the cryopreservation period;
FIG. 7-1 is a view of optical microscope photographs each showing a form of an aggregate before freezing and after thawing of proximal tubular epithelial cells frozen in a dispersed state (Comparative Example);

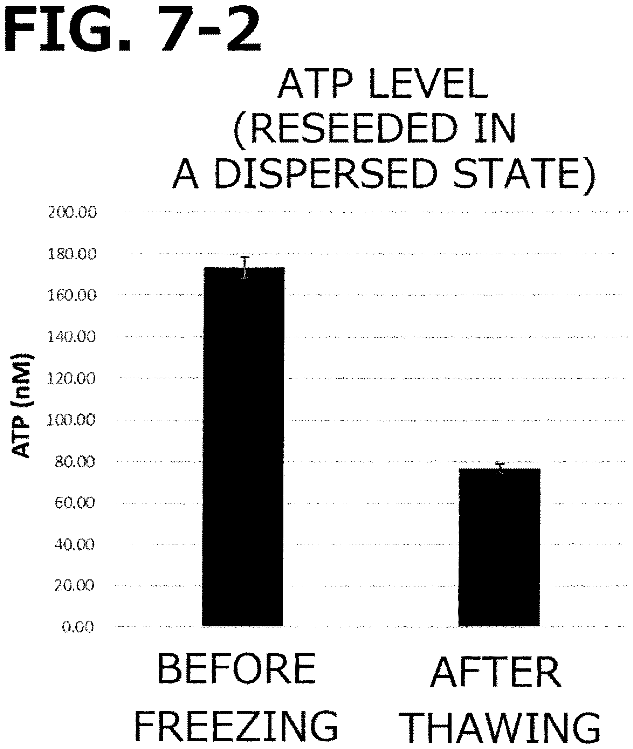

FIG. 7-2 is a diagram illustrating ATP levels (cell viability) before freezing and after thawing of proximal tubular epithelial cells frozen in a dispersed state (Comparative Example); and

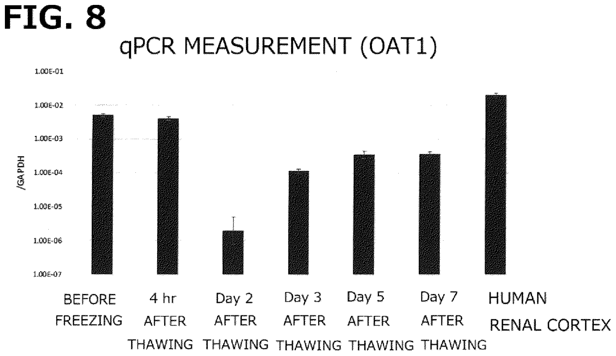

FIG. 8 is a diagram illustrating, in comparison with human renal cortex, results of OAT1 gene expression analysis by a real-time PCR method of proximal tubular epithelial cells frozen in a dispersed state (Comparative Example).

### DETAILED DESCRIPTION

The present inventors have found that aggregates of renal cells are frozen in an aggregated state, whereby it is possible to stably cryopreserve the aggregates while maintaining the cell viability and the form, and further, it is possible to obtain a renal cell culture in which the functions of the kidney after thawing are maintained from the aggregates cryopreserved in an aggregated state. Thus, they have completed the present invention.

The method of producing frozen-state renal cells in accordance with the methods of the present invention includes a recovery step of recovering cultured renal cells and a freezing step of freezing the renal cells recovered in the recovery step. In this recovery step, the renal cells are recovered as an aggregate. Further, in the freezing step, the aggregate is frozen while an aggregated state of the aggregate is substantially maintained.

The renal cells used in the present invention may be any source as long as the renal cells can be cultured. The renal cells are preferably derived from mammals, and are preferably derived from primates such as humans and monkeys. Depending on the purpose, the renal cells may be derived from normal kidney or kidney having a disease. Examples of renal cells include cells constituting epithelium, cortex, proximal tubule, distal tubule, collecting duct, glomerulus, and the like, specifically, proximal tubular epithelial cells (RPTEC), and mesangial cells. The renal cells may be primary cells or renal cells derived from stem cells such as iPS cells or ES cells. Further, the renal cells may be immortalized renal cells, established cells (such as HK-2 cells), cells derived from other animal species (such as MDCK cells, LLC-PK1 cells, and JTC-12 cells), and forcibly expressed cells obtained by introducing genes into renal cells in order to express proteins such as specific transporters. More specific examples of renal cells include human proximal tubular epithelial cells, human distal tubular epithelial cells, and human collecting tubular epithelial cells collected and isolated from the kidney; and proximal tubular epithelial cells, distal tubular epithelial cells, and collecting tubular epithelial cells induced to differentiate from human iPS cells or human ES cells. In drug discovery research, it is preferable to use proximal tubular epithelial cells, particularly proximal tubular epithelial cells derived from human normal kidney.

Renal cells can be cultured using a culture medium and a culture vessel suitable for the cells to be cultured according to an ordinary method, for example, under conditions of 37°C and 5% CO₂. The culture may be any of static culture, shaking culture, stirring culture, and the like. The culture may be adhesion culture, but it is preferable to perform culture in a state of being non-adhered (e.g., suspension culture) for at least a part of the period. Culturing of renal cells in a state of being non-adhered onto a culture vessel allows for formation of aggregates. The "state of being non-adhered" refers to a state in which all or most of the cells are not adhered to the surface of the culture vessel, and includes a state in which all or most of the cells are separated from the surface of the culture vessel, and a state in which even when the cells are in contact with the surface of the culture vessel, if the cells could be easily separated from the surface of the culture vessel by coating of the culture vessel, convection of the culture medium, or the like without using a tool, an enzyme, or the like.

For example, in some cases, aggregates of renal cells are formed on Day 1 of renal cell culture (i.e., within 24 hours). Then, renal cells in an aggregated state are cultured for a part of the period, so that it is possible to restore the physiological function of renal cells that have been reduced by dedifferentiation. Desirably, the period during which renal cells are cultured in a state of being non-adhered onto a culture vessel is commonly 5 days or more (i.e., 120 hours or more). This makes it possible to obtain cultured renal cells in a state of higher expression of physiological functions of the kidney. During the culture period, the culture medium is preferably replaced periodically. For example, the culture medium is replaced every two days.

As the medium, any known culture medium can be appropriately used. For example, in the case of culturing proximal tubular epithelial cells, a commercially available tubular cell culture medium can be used, and examples of preferred media include REGM (registered trademark) (Lonza), EpiCM (registered trademark) (ScienCell Research Laboratories, Inc.), and KeratinocyteSFM (registered trademark) (Thermo Fisher Scientific, Inc.).

In addition, conventionally known materials and additives useful for cell culture can be appropriately used. For example, collagen I (type I collagen) can be added to the culture medium. Collagen I has an action of causing renal cells to attach to each other. Thus, the formation of aggregates is promoted by culturing renal cells in a culture medium containing collagen I. Collagen I is preferably full-length collagen I, and may be an α1 chain or an α2 chain of collagen I, a collagen peptide obtained by fragmenting each of the chains, or the like. The source of collagen I is not particularly limited, and collagen I may be derived from humans or other animals.

Any culture vessel can be used. In order to promote the formation of cell aggregates, it is preferable that the culture vessel is subjected to a cell non-(low) adhesion process or is made of a cell non-(low) adhesive material. Examples of cell non-(low) adhesion processes include a cell non-adhesive hydrogel coating process, a 2-methacryloyloxyethyl phosphorylcholine (MPC) coating process, a Proteosave (registered trademark) SS coating process, and a mirror polishing process to the surface of the vessel. Examples of cell non-(low) adhesive materials include glass and polymeric materials such as low-density polyethylene, medium-density polyethylene, polyvinyl chloride, polyethylene-vinyl acetate copolymer, poly(ethylene-ethylacrylate) copolymer, poly(ethylene-methacrylate) copolymer, and poly(ethylene-vinylacetate) copolymer, and a mixture of two or more of these polymers.

In a case where a large number of aggregates are formed, it is possible to use a plate or dish for producing high-density spheroid. Furthermore, a culture vessel such as a spinner flask may be used as necessary. For example, it is preferable to use a culture vessel of ELPLASIA (registered trademark) series (Corning Incorporated.), a culture vessel of EZSPHERE (registered trademark) series (AGC TECHNO GLASS Co.,Ltd.), or the like. These culture vessels are of types such as 6-well plates, 24-well plates, 96-well plates, 384-well plates, and dishes of various sizes. The number of spheroids or aggregates that can be produced varies depending on the size of the area of the vessel bottom. For example, in the case of using a 96-well plate (V-bottom), a 96-well plate (U-bottom), or a 384-well plate (U-bottom), each of which has been subjected to a low adhesion process, one cell aggregate is formed in one well.

Aggregates produced using a plate or dish for producing high-density spheroid or the like can be recovered and shake-cultured in a suspended state. In the case of shake-culturing in a suspended state, it is preferable that a dish, a plate, or the like subjected to a cell non-(low) adhesion process is placed on a shaker, and aggregates are cultured. A reciprocating shaker and a swirling shaker can be used as the shaker.

The term "an aggregate" of cells used herein refers to a massive aggregate of several or more cells. The number of cells constituting the aggregate is, for example, 5 or more, 25 or more, 50 or more, preferably 100 or more, and more preferably 125 or more. The number of cells constituting the aggregate is, for example, 10,000 or less, preferably 5000 or less, more preferably 2000 or less, or 1000 or less. When the number of cells constituting the aggregate is too small, the size of the aggregates is reduced, and there is a possibility that variations in aggregate production are increased. Further, there is a tendency that the aggregates are bonded to each other and the size is less likely to be uniform. Meanwhile, when the number of cells constituting the aggregate is too large, the characteristic gene expression level of renal cells may be lowered, and there is a tendency that it is difficult to maintain the gene expression level after cryopreservation of the aggregate.

The size of the aggregates can be controlled by adjusting the number of cells seeded in the culture vessel. For example, in the case of culturing in a multi-well plate, one aggregate is formed in one well. Thus, when the number of seeded renal cells per well is 500 or more and 5000 or less, the number of renal cells constituting the aggregate is 500 or more and 5000 or less. The size of the aggregates is 100 µm or more and 350 µm or less when the number of constituent cells is 500 or more and 5000 or less.

In the case of culturing using a culture vessel such as a dish or a spinner flask, the diameter of aggregates or the number of renal cells constituting one aggregate can be controlled by adjusting the density of the cells in the vessel. For example, in the case of forming aggregates whose diameter is about 100 µm or more and about 350 µm or less or in which the number of constituent cells is 500 or more and 5000 or less, the density of the cells in the vessel can be adjusted to 1500 cells/cm² or more and 15000 cells/cm² or less.

The size of an aggregate is defined as the maximum width of the aggregate. That is, the size of the aggregate is the length of the longest one of straight lines connecting two points on the outer edge of the aggregate. Since the aggregate is approximately spherical, hereinafter, the size of the aggregate may be appropriately referred to as the diameter of the aggregate for convenience.

The renal cells thus cultured can be recovered in the recovery step. The phrase "recovered as an aggregate" means that the renal cells are recovered without any operation that intentionally breaks or disperses the formed aggregate (e.g., trypsinization), in order not to impair the form of the aggregate. Therefore, the recovered renal cells only need to contain cells in an aggregated form, and there may be cells in a dispersed state, such as cells that have formed no aggregate during culture or cells that have been accidentally detached from an aggregate.

In order to recover aggregates, a wide mouth tip is preferably used so as not to damage the aggregated form. In the wide mouth tip, the diameter of the tip is wider than that of a normal tip. A tip having an inner diameter larger than the diameter of the aggregates, for example, an inner diameter of about 1.5 mm (1500 µm) is used, so that renal cells can be recovered without damaging the form of the aggregates. As an operation example of the recovery step, first, aggregates are sucked together with a culture medium using a wide mouth tip and transferred to a centrifuge tube. The centrifuge tube is centrifuged (at 160 g for 3 min), and the aggregates are collected to the bottom of the centrifuge tube. Alternatively, the culture medium containing aggregates may be transferred to a 1.5 mL tube or the like, and the aggregates may be collected by natural sedimentation. When freezing the aggregates thus recovered, it is preferable that the supernatant is removed by suction and a cryopreservation medium containing a cryoprotectant described later is added.

It is possible to freeze the recovered renal cells by cooling the renal cells under low temperatures at which the renal cell can freeze, until the renal cells freeze. Freezing of renal cells is preferably performed by slow freezing. Slow freezing refers to a method in which cells are frozen while the rate of temperature drop of the cells to be frozen is adjusted to a predetermined range so that the cells are gradually frozen. The rate of temperature drop can be adjusted by utilizing a commercially available cryopreservation vessel, a program freezer capable of setting freezing conditions of cells and tissues, or the like. Examples of cryopreservation vessels used for slow freezing include cryopreservation vessels such as BICELL (registered trademark) (Nippon Freezer Co., Ltd.) and CoolCell (registered trademark) (Corning Incorporated.), and a cryopreservation vessel in which slow freezing is performed using isopropyl alcohol. The rate of temperature drop of cells during freezing in slow freezing can range, for example, from about 0.2°C to about 3°C per minute, and is preferably about 1°C per minute.

Cooling of renal cells can be performed using a common freezer. For example, when the aggregates of proximal tubular epithelial cells are frozen, it is preferable to use an ultra-low temperature freezer that can be set to a temperature of - 80°C.

The phrase "an aggregated state of the aggregate is substantially maintained" in the freezing step means that the form of the aggregate is prevented from being impaired by not performing any operation that intentionally breaks or disperses the aggregate contained in the recovered renal cells (e.g., trypsinization). Further, the term "substantially" means that a decrease in the aggregate number after freezing is within a range that does not cause a problem as compared with the aggregate number in the recovered renal cells before freezing, and does not necessarily mean that the cells constituting the aggregate are not dispersed at all during freezing.

The method of this embodiment may include an optional step between the recovery step and the freezing step, such as other steps that are beneficial for the cryopreservation of the cells, as long as they do not significantly adversely affect the maintenance of the form of the aggregate. It is possible to include, for example, a step of adding a suitable cryopreservation medium to the recovered renal cells.

Examples of cryopreservation media include media containing a cryoprotectant component that reduces damage from intracellular ice crystals, such as any of dimethylsulfoxide (5 to 15%), mammalian serum, dextran, glycogen, methylcellulose or carboxymethyl cellulose, polyethylene glycol, polyvinylpyrrolidone, glucose, and sucrose. The medium can be a liquid, such as a culture medium. A solution in which two or more of these cryoprotectants are appropriately blended is preferable. Therefore, as the cryopreservation medium, a commercially available cryopreservation solution such as CELLBANKER (registered trademark) 1 plus (ZENOAQ RESOURCE CO., LTD.), CELLBANKER (registered trademark) 1 (ZENOAQ RESOURCE CO., LTD.), or CELLBANKER (registered trademark) 2 (ZENOAQ RESOURCE CO., LTD.) may be used.

The frozen renal cells can be preserved using a common freezer. For example, when the aggregates of proximal tubular epithelial cells are preserved, it is preferable to use an ultra-low temperature freezer that can be set to a temperature of - 80°C. In a case where the cells are cryopreserved for a longer period of time, it is preferable to preserve the cells in a lower temperature environment, for example, a liquid nitrogen storage vessel (-150°C or less).

Frozen state renal cells can be thawed by a known method. For example, a frozen vial can be thawed in a thermostat bath at 37°C. After thawing, the renal cells can be cultured in the same manner as described above for culturing for aggregate formation. For example, thawed renal cells are mixed with a culture medium and centrifuged (160 × g, 3 min), and the supernatant is removed. A necessary amount of culture medium is added to the collected renal cells, and the cells are transferred to a culture vessel such as a culture dish or a culture plate and cultured. In a case where a large number of aggregates are cultured, the aggregates are transferred to a low adhesion dish, and the aggregates are shake-cultured in a suspended state. The thawed renal cells are preferably cultured in a state of being non-adhered to the culture vessel for 5 days or longer, more preferably 7 days or longer after thawing. During the culture period, the culture medium is preferably replaced periodically. As described above, a renal cell culture having higher renal physiological functions or being functionally equivalent compared with human renal cortex can be produced from cryopreserved renal cells at desired time points.

The phrase "being functionally equivalent compared with human renal cortex" means that regarding expression of at least one or more genes associated with physiological functions of the kidney expressed in the human renal cortex, a level of the gene expression equivalent to the human renal cortex is exhibited.

Examples of genes related to physiological functions of the kidney include AQP1, CD13, SGLT2, Na/K ATPase, URAT1, PEPT1, MDR1, OAT1, OCT2, OCTN2, E-cadherin, and ZO-1. AQP1 (aquaporin 1) is a gene encoding for a protein involved in water transport. CD 13 (alanyl aminopeptidase) is a gene coding for a protein involved in peptidization of protein. SGLT2 (sodium glucose cotransporter 2) is a gene coding for a protein involved in sodium and glucose transport. Na/K ATPase is a gene coding for a protein involved in ion transport. URAT1 (urate transporter 1) is a gene coding for a protein involved in uric acid reabsorption. PEPT1 (peptide transporter 1) is a gene coding for a protein involved in peptide transport. MDR1 (multiple drug resistance 1), OAT1 (organic anion transporter 1), OCT2 (organic cation transporter 2), and OCTN2 (organic cation transporter novel 1) are genes coding for proteins involved in drug transport. E-cadherin and ZO-1 (zonula occludens-1) are genes coding for proteins involved in intercellular junction.

The expression levels of one or more of these genes in the human renal cortex and the renal cell culture can be measured by a common real-time PCR method (qPCR method), and whether or not both the expression levels are equivalent can be determined by comparing them. When used for determination, measured values of two or more experiments are averaged and used.

For example, in a case where frozen renal cells are cultured for 7 days after thawing, and the expression level of any of the above genes is 10% or more of the expression level of the gene in the human renal cortex, the renal cell culture is determined to be functionally equivalent to the human renal cortex. In the renal cell culture functionally equivalent to the human renal cortex, the expression level of one of the above genes is preferably 10% or more, more preferably 25% or more of the expression level of the gene in the human renal cortex. Alternatively, in the renal cell culture functionally equivalent to the human renal cortex, the expression levels of two or more of the above genes are preferably 10% or more, more preferably 25% or more of the expression levels of the genes in the human renal cortex. Such expression levels in the renal cell culture of this embodiment are significantly higher than expression levels in conventional two-dimensional cultured renal cell cultures. The expression levels of, particularly OAT1 and OCT2 in the renal cell culture can be comparable to those in the human renal cortex. Therefore, in the renal cell culture functionally equivalent to the human renal cortex, the expression level of one or both of OAT1 and OCT2 is preferably 40% or more, more preferably 55% or more of the expression level thereof in the human renal cortex.

Similarly, the expression levels of one or more of the genes in the renal cells before freezing are compared with the expression levels of one or more of the genes in the renal cell culture after thawing, so that it is possible to determine whether both the renal cells and the renal cell culture are functionally equivalent.

For example, in a case where frozen renal cells are cultured for 7 days after thawing, and the expression level of any of the above genes is 40% or more of the expression level of the gene in the renal cells before freezing, the renal cell culture is determined to be functionally equivalent to the kidney. In the renal cell culture functionally equivalent to the kidney, the expression level of one of the above genes is preferably 40% or more, more preferably 55% or more of the expression level of the gene in the renal cells before freezing. Alternatively, in the renal cell culture functionally equivalent to the kidney, the expression levels of two or more of the above genes are preferably 40% or more, more preferably 55% or more of the expression levels of the genes in the renal cells before freezing.

The cell viability after thawing and physiological functions of the kidney can be well maintained by the production method of this embodiment including freezing renal cells such as proximal tubular epithelial cells in an aggregated state, which is better than by using a method of freezing renal cells as a cell suspension in the conventional art. According to this embodiment, it is possible to cryopreserve renal cell aggregates while maintaining renal functions and cell viability of the aggregates in which the renal functions are lost by two-dimensional culture are restored by three-dimensional culture, and it is possible to provide a renal cell product and a renal cell culture which can be utilized in drug discovery research.

The present invention is defined by te claims.

### Examples

### 1. Culture of Renal Cells and Formation and Freezing of Aggregates

Human proximal tubular epithelial cells (Clonetics (registered trademark), Cat # CC-2553, RPTEC-Renal proximal tubular epithelial cells) obtained from Lonza were used as renal cells. The cells were thawed according to the manufacturer's instructions and cultured using the recommended medium (REGM (registered trademark), Lonza) under conditions of 37°C and 5% CO₂ with culture medium change once every two days. The cells were recovered before becoming confluent and cultured in a 96-well V-bottom plate (PrimeSurface (registered trademark) Plate 96V, Sumitomo Bakelite Co., Ltd.) which had been subjected to a cell low adhesion process, thereby forming aggregates. The aggregates were cultured while changing the culture medium once every two days. The term "confluent" means that the ratio of the area occupied by the cells to the entire culture surface of the culture vessel is about 100%, i.e., a state in which the cells have grown to the full extent of the culture surface without gaps.

After culturing for 10 days or longer, the culture solution containing aggregates was recovered and dispensed into a frozen vial so that about 1000 aggregates were contained per vial. 500 µL/vial of a cryopreservation solution (CELLBANKER (registered trademark) 1 plus, ZENOAQ RESOURCE CO., LTD.) was added to the aggregates after removal of the culture medium, and the mixture was well mixed. Thereafter, the aggregates were placed in a cryopreservation vessel (BICELL (registered trademark), Nippon Freezer Co., Ltd.) and slowly frozen in an ultra-low temperature freezer at -80°C.

The produced frozen cells were cryopreserved for one week or longer, thawed, and shake-cultured in a suspended state for 7 days while changing the culture medium once every two days.

The cell viability of the cells after culture was measured using CellTiter-Glo (registered trademark) 3D Cell Viability Assay (Promega Corporation) for measuring the ATP level by a luminescence method. Specifically, aggregates were collected together with the culture medium, and CellTiter-Glo 3D Reagent was added in an amount equal to the volume of the culture medium. The mixture was incubated at room temperature for 30 minutes. The mixture was well mixed, and then the luminescence value was measured with a microplate reader (Perkin Elmer).

FIG. 1-1 shows the form of the thawed aggregates observed with an optical microscope. "Day 0 after thawing" represents the day of thawing, "Day 1 after thawing" represents the day after thawing, and "Day 5 after thawing" represents the fifth day after thawing (the number of days from the first thawing is similarly described in the following experiments). FIG. 1-2 shows the results of ATP level measurement. "Before freezing" represents a time point before freezing of aggregated cells cultured for 10 days or longer, and "After thawing" represents a time point when aggregates are frozen for 1 week or longer, thawed, and shake-cultured in a suspended state for 7 days.

The proximal tubular epithelial cells frozen in an aggregated state were confirmed to maintain the aggregate form after thawing. As a result of measuring the ATP level, it was confirmed that most of the cells survived even after thawing, and good cell viability was maintained.

### 2. Analysis of Gene Expression Level

Gene expression in proximal tubular epithelial cell aggregates before and after freezing was examined and compared with gene expression in human renal cortex.

Aggregates of renal cells were formed, and the aggregates were frozen in an aggregated state in the same manner as in 1. above. Thereafter, the cells were thawed, and the resultant cells were shake-cultured in a suspended state while changing the culture medium once every two days.

mRNA was extracted and purified from aggregates before freezing and after thawing and culturing for a predetermined period using RNeasy (registered trademark) Mini Kit (QIAGEN). Further, cDNA was synthesized from this mRNA using QuantiTect (registered trademark) Whole Transcriptome Kit (QIAGEN). The synthesized cDNA was used as a template, the gene expression levels of OAT1 (organic anion transporter 1), OCT2 (organic cation transporter 2), and URAT1 (uric acid transporter 1) abundantly expressed in the proximal tubule were measured by a real-time PCR method using Thermal Cycler Dice (registered trademark) Real Time System 1 (Takara Bio Inc.). In addition, the gene expression level of OAT1 was measured for aggregates thawed and shake-cultured in a suspended state for 2 days, 4 days, or 7 days. For all experiments, each sample was measured with n = 3 in one experiment.

For comparison, RNA was extracted in the same manner as described above using human renal cortex collected from human patient donors, and the gene expression level of OAT1, OCT2, or URAT1 was measured.

FIGS. 2-1, 2-2, 2-3, and 2-4 show the results. FIG. 2-1 shows the results of the gene expression level of OAT1, FIG. 2-2 shows the results of the gene expression level of OCT2, and FIG. 2-3 shows the results of the gene expression level of URAT1. In FIGS. 2-1, 2-2, and 2-3, "Before freezing" represents a time point before freezing of aggregated cells cultured for 10 days or longer, and "After thawing" represents a time point when aggregates are frozen for 1 week or longer, thawed, and shake-cultured in a suspended state for 7 days. FIG. 2-4 shows the results of gene expression levels of aggregates thawed and shake-cultured in a suspended state for 2 days, 4 days, or 7 days.

In addition, comparison between gene expression in aggregates before freezing and gene expression in human renal cortex (Table 1-1), comparison between gene expression in aggregates after thawing and gene expression in human renal cortex (Table 1-2), and comparison between gene expression in aggregates before freezing and gene expression in aggregates after thawing (Table 2) are shown (measured values of two experiments and the average value of these measured values). In Tables 1-1, 1-2, and 2, "Before freezing" represents a time point before freezing of aggregated cells cultured for 10 days or longer, and "After thawing" represents a time point when aggregates are frozen for 1 week or longer, thawed, and shake-cultured in a suspended state for 7 days.

**[Table 1-1]**

| (GAPDH ratio) | First experiment (Aggregates before /human renal cortex) | Second experiment (Aggregates before freezing /human renal cortex) | Average value (Aggregates before freezing /human renal cortex) |
|---|---|---|---|
| OAT1 | 85.9% | 59.9% | 72.9% |
| OCT2 | 280.2% | 71.0% | 175.6% |
| URAT1 | 6.7% | 7.5% | 7.1% |

**[Table 1-2]**

| (GAPDH ratio) | First experiment (Aggregates after thawing /human renal cortex) | Second experiment (Aggregates after thawing /human renal cortex) | Average value (Aggregates after thawing /human renal cortex) |
|---|---|---|---|
| **OAT1** | **161.7%** | **90.5%** | **126.1%** |
| **OCT2** | **157.6%** | **76.3%** | **117.0%** |
| **URAT1** | **9.8%** | **13.5%** | **11.6%** |

**[Table 2]**

| (GAPDH ratio) | First experiment (Aggregates after thawing /aggregates before freezing) | Second experiment (Aggregates after thawing /aggregates before freezing) | Average value (Aggregates after thawing /aggregates before freezing) |
|---|---|---|---|
| **OAT1** | **188.3%** | **151.1%** | **169.7%** |
| **OCT2** | **56.2%** | **107.4%** | **81.8%** |
| **URAT1** | **147.1%** | **179.4%** | **163.2%** |

When renal cells were frozen in an aggregated state, the operations of freezing and thawing did not affect the gene expression levels of OAT1, OCT2, and URAT1 in the renal cells. When the thawed renal cells were shake-cultured in a suspended state for 7 days, the thawed renal cells exhibited a gene expression level equivalent to that of human renal cortex. Therefore, it was confirmed that the physiological functions of the proximal tubular epithelial cells frozen in an aggregated state were well maintained, and a cell culture equivalent to that of human renal cortex was obtained by culturing the cells after thawing.

### 3. Influence of Number of Cells in Aggregate

Aggregates of renal cells were formed, and the aggregates were frozen in an aggregated state in the same manner as in 1. above. However, when aggregates were formed, the number of cells per aggregate was adjusted to 125, 250, 500, or 1000.

mRNA was extracted and purified from aggregates before freezing and after thawing and shake-culturing in a suspended state for 2 days, 5 days, or 7 days, cDNA was synthesized, and the gene expression level of OAT1 was measured in the same manner as in the above 2.

FIG. 3 shows the results of the gene expression levels. The thawed aggregates with a number of cells of 125 to 1000 were cultured for 7 days, as a result of which in any number of cells, the expression level of OAT1 was equivalent to that in the aggregates before freezing and also equivalent to that in the human renal cortex. On Day 2 after thawing, it was observed that the expression level of OAT1 tended to be more favorably maintained as the number of cells in each aggregate was smaller, but it was observed that even in the case of aggregates having a large number of cells, the expression level could be restored by extending the culture period. Therefore, concerning the aggregates with a wide range of number of cells, it was confirmed that a good cell culture was obtained after thawing.

### 4. Influence of Freezing Method

Aggregates of renal cells were formed, and the aggregates were frozen in an aggregated state in the same manner as in 1. above. However, at the time of freezing, the aggregates were not slowly frozen in a cryopreservation vessel (BICELL (registered trademark), Nippon Freezer Co., Ltd.), and 20 µL/well of a cryopreservation solution (CELLBANKER (registered trademark) 1 plus, ZENOAQ RESOURCE CO., LTD.) was added to the aggregates after removal of the culture medium as they were in a 96-well plate. Then, the resultant mixture was well mixed and frozen in an ultra-low temperature freezer.

The cells were thawed and cultured for 7 days with culture medium change once every two days. The ATP level in the cells was measured in the same manner as in 1. above, and the cell viability was determined. From the thawed aggregates, mRNA was extracted and purified in the same manner as in 2. above, cDNA was synthesized, and the gene expression level of OAT1 was measured.

FIG. 4-1 shows the results of ATP level measurement. FIG. 4-2 shows the results of the gene expression levels. In FIGS. 4-1 and 4-2, "Before freezing" represents a time point before freezing of aggregated cells cultured for 10 days or longer, and "After thawing" represents a time point when aggregates are frozen for 1 week or longer, thawed, and shake-cultured in a suspended state for 7 days.

Aggregates of proximal tubular epithelial cells exhibited lower cell viability in the case of the method without slow freezing, as compared with the case of slow freezing. Also, when slow freezing was not performed, aggregates after freezing exhibited reduced OAT1 expression as compared with when slow freezing was performed. Therefore, it was confirmed that when the aggregates of proximal tubular epithelial cells were frozen in an aggregated state, slow freezing was advantageous in terms of maintaining cell viability and maintaining renal functions.

### 5. Influence of Cryopreservation Solution

Aggregates of renal cells were formed, and the aggregates were frozen in an aggregated state in the same manner as in 1. above. However, 500 µL/vial of a cryopreservation solution (CELLBANKER (registered trademark) 1 plus, ZENOAQ RESOURCE CO., LTD.) or REGM containing 10% dimethylsulfoxide (manufactured by SIGMA CORPORATION) was added to the aggregates after removal of the culture medium, and the mixture was well mixed. Thereafter, the aggregates were placed in a cryopreservation vessel (BICELL (registered trademark), Nippon Freezer Co., Ltd.) and slowly frozen in an ultra-low temperature freezer at -80°C.

Respective cells were thawed and shake-cultured in a suspended state while changing the culture medium once every two days. The ATP level in the cells in the aggregates 7 days after thawing was measured in the same manner as in 1. above, and the cell viability was determined.

FIG. 5 shows the results of ATP level measurement.

As compared with aggregates frozen in REGM containing 10% dimethylsulfoxide, aggregates frozen in CELLBANKER (registered trademark) 1 plus exhibited high cell viability after thawing. Therefore, the addition of a cryopreservation solution containing two or more cryoprotectant components was confirmed to be advantageous.

### 6. Influence of Freezing Period

Aggregates of renal cells were formed, and the aggregates were frozen in an aggregated state in the same manner as in 1. above. The cells were cryopreserved in an ultra-low temperature freezer at -80°C for 1 week and cryopreserved for 5 months or longer, and then thawed. In the same manner as in 2. above, the thawed cells were shake-cultured in a suspended state for 7 days while changing the culture medium once every two days. From the aggregates 7 days after thawing, mRNA was extracted and purified in the same manner as in 2. above, cDNA was synthesized, and the gene expression level of OAT1 was measured.

FIG. 6 shows the results of the gene expression levels.

The expression levels of OAT1 observed in both short-term (1 week) freezing and long-term (5 months or longer) freezing were equivalent to each other. Therefore, it was confirmed that the renal cells could be stably cryopreserved at -80°C for a period of at least several months or longer while maintaining physiological functions of the kidney.

### 7. Cell viability of Renal Cells Frozen in Dispersed State (Comparative Example)

In a case where a cell suspension obtained by dispersing the same renal cells as those in 1. with a digestive enzyme was frozen (conventional common method for freezing cells), the cell form and the survival rate were examined.

Proximal tubular epithelial cells were thawed according to the manufacturer's instructions and cultured using the REGM under conditions of 37°C and 5% CO₂, with culture medium change once every two days. The cells were dispersed and recovered using Accutase (registered trademark) (Innovative Cell Technologies, Inc.) before becoming confluent. The number of cells was measured and the necessary amount was centrifuged to remove the culture medium. CELLBANKER (registered trademark) 1 plus (ZENOAQ RESOURCE CO., LTD.) was added to the cells so that the cell concentration was 5 × 10⁵ cells/mL or more to form a cell suspension. The cell suspension was dispensed into frozen vials. The vials were placed in a cryopreservation vessel (BICELL (registered trademark), Nippon Freezer Co., Ltd.) and slowly frozen in an ultra-low temperature freezer at -80°C. After cryopreservation for a certain period, cells were thawed, diluted with the REGM to a cell count of 1000, and seeded into a low attachment 96-well V-bottom plate (PrimeSurface (registered trademark) plate 96V, Sumitomo Bakelite Co., Ltd.). After seeding, the cells were cultured while changing the culture medium once every two days.

In addition, cells before freezing and cells after thawing were seeded so as to have the same number of cells, and cultured. After seeding, the culture medium was changed once every two days, and the ATP level (cell viability) was measured from aggregates 7 days after thawing using CellTiter-Glo (registered trademark) 3D Cell Viability Assay (Promega Corporation).

FIG. 7-1 shows the form of the cells after thawing observed with an optical microscope. FIG. 7-2 shows the results of ATP level measurement.

As shown in FIG. 7-1, the renal cells had formed an aggregate of 1000 cells before freezing. When this aggregate was dispersed and frozen as a cell suspension in the conventional manner, no aggregate was formed even when the cells after thawing were seeded on a low attachment plate. Further, when the same number of cells were seeded, cells frozen in the cell suspension exhibited lower cell viability as compared with those before freezing. Therefore, it was confirmed that in the conventional common freezing method, the survival rate of aggregates was reduced, and functions such as cell-cell adhesion were impaired, whereby no aggregate could be formed.

### 8. Gene Expression Analysis of Renal Cells Frozen in Dispersed State (Comparative Example)

When a cell suspension obtained by dispersing the same renal cells as those in 1. with a digestive enzyme was frozen (conventional common cell freezing method), the gene expression level was examined.

A cell suspension of proximal tubular epithelial cells was frozen in the same manner as in the above 7. and thawed, the cells were seeded so as to form an aggregate of 1000 cells. After seeding, the cells were cultured while changing the culture medium once every two days. mRNA was extracted and purified over time from cells before freezing and after thawing using RNeasy (registered trademark) Mini Kit (QIAGEN), and cDNA was further synthesized using QuantiTect (registered trademark) Whole Transcriptome Kit (QIAGEN). These synthesized cDNA was used as a template, the gene expression level of OAT1 was measured by a real-time PCR method using Thermal Cycler Dice (registered trademark) Real Time System 1 (Takara Bio Inc.).

FIG. 8 shows the results of the gene expression levels.

The renal cells before freezing exhibited good OAT1 expression. However, when the renal cells were frozen as a cell suspension in the conventional manner, even after 7 days of culture after thawing, the expression of OAT1 in the resultant cells was lower compared with the expression of OAT1 before freezing and the expression of OAT1 in human renal cortex. Therefore, freezing of aggregates of proximal tubular epithelial cells in an aggregated state was confirmed to be more advantageous in terms of maintaining physiological functions of the kidney, compared with the conventional common cell freezing method.

### (Embodiments to be used in accordance with the present invention)

A first embodiment is a method of producing frozen-state renal cells, including: a recovery step of recovering cultured renal cells; and a freezing step of freezing the renal cells recovered in the recovery step, in which the renal cells are recovered as an aggregate in the recovery step, and the aggregate is frozen while an aggregated state of the aggregate is substantially maintained in the freezing step. Accordingly, an effect is exerted such that frozen renal cells having good aggregate form and cell viability after thawing can be produced.

A second embodiment is, in the first embodiment, to further include a step of adding a cryopreservation medium to the recovered renal cells between the recovery step and the freezing step. Accordingly, the effect of frozen renal cells exhibiting good cell viability after thawing is enhanced.

A third embodiment is that, in the first or second embodiment, the number of renal cells constituting the aggregate is 100 or more and 5000 or less. Accordingly, the renal cell culture obtained from the frozen renal cells exhibits an effect of maintaining physiological functions of the kidney well.

A fourth embodiment is that, in any one of the first to third embodiments, the renal cells are further frozen by slow freezing in the freezing step. Accordingly, an effect is exerted such that the frozen renal cells exhibit good cell viability after thawing and maintain physiological functions of the kidney well.

A fifth embodiment is that, in any one of the first to fourth embodiments, the cultured renal cells include aggregates obtained by culturing renal cells in a state of being non-adhered onto a culture vessel. Accordingly, an effect is exerted such that the frozen renal cells exhibit good cell viability after thawing and maintain physiological functions of the kidney well.

A sixth embodiment is that, in any one of the first to fifth embodiments, the renal cells are human primary cells. Accordingly, it is easy to reflect a clinical phenomenon in humans, and an effect of being suitable for use in drug discovery research for developing human drugs is exerted.

A seventh embodiment is that, in any one of the first to sixth embodiments, the renal cells are proximal tubule-derived cells. Accordingly, the renal cells and the renal cell culture easily reflect the influence of the drug on the kidney, and an effect of being suitable for use in the evaluation of pharmacokinetics and nephrotoxicity of the drug is exerted.

An eighth embodiment includes frozen-state renal cells produced by the method of any one of the first to seventh embodiments. Accordingly, an effect is exerted such that a cell culture with well-maintained physiological functions of the kidney can be easily obtained by culturing after thawing.

An embodiment of the present invention is a method as claimed of producing a renal cell culture including a culturing step of thawing frozen-state renal cells produced by the method according to any one of the first to seventh embodiments and culturing the renal cell in a state of being non-adhered onto a culture vessel. Accordingly, an effect is exerted such that a renal cell culture with well-maintained physiological functions of the kidney and usable for drug discovery research can be easily produced when necessary.

### Industrial Applicability

The present invention can be used in the production and preservation of renal cells that can be used in drug discovery research and the like.

## Claims

1. A method of producing a renal cell culture comprising:
a recovery step of recovering cultured renal cells; and
a freezing step of freezing the renal cells recovered in the recovery step, and
a culturing step of thawing frozen-state renal cells,
wherein:
the renal cells are recovered as an aggregate in the recovery step, and
the aggregate is frozen while an aggregated state of the aggregate is maintained in the freezing step, and
the renal cells are cultured in a state of being non-adhered onto a culture vessel for 5 days or longer in the culturing step.

2. The method according to claim 1, further comprising a step of adding a cryopreservation medium to the recovered renal cells between the recovery step and the freezing step.

3. The method according to claim 1 or 2, wherein a number of the renal cells constituting the aggregate is 100 or more and 5000 or less.

4. The method according to any one of claims 1 to 3, wherein the renal cells are frozen by slow freezing in the freezing step.

5. The method according to any one of claims 1 to 4, wherein the cultured renal cells include aggregates obtained by culturing renal cells in a state of being non-adhered onto a culture vessel.

6. The method according to any one of claims 1 to 5, wherein the renal cells are human primary cells.

7. The method according to any one of claims 1 to 6, wherein the renal cells are proximal tubule-derived cells.

## Patentansprüche

1. Verfahren zur Herstellung einer Nierenzellkultur, umfassend:
einen Gewinnungsschritt der Gewinnung von kultivierten Nierenzellen; und
einen Gefrierschritt des Einfrierens der im Gewinnungsschritt gewonnenen Nierenzellen, und
einen Kultivierungsschritt des Auftauens von im gefrorenen Zustand befindlichen Nierenzellen,
wobei:
die Nierenzellen in dem Gewinnungsschritt als ein Aggregat gewonnen werden, und
das Aggregat eingefroren wird, während ein aggregierter Zustand des Aggregats in dem Gefrierschritt beibehalten wird, und
die Nierenzellen in dem Kultivierungsschritt in einem Zustand, in dem sie nicht an einem Kulturgefäß haften für 5 Tage oder länger kultiviert werden.

2. Verfahren gemäß Anspruch 1, weiter umfassend einen Schritt der Zugabe eines Kryokonservierungsmediums zu den gewonnenen Nierenzellen zwischen dem Gewinnungsschritt und dem Gefrierschritt.

3. Verfahren gemäß Anspruch 1 oder 2, wobei eine Anzahl der Nierenzellen, die das Aggregat bilden, 100 oder mehr und 5000 oder weniger beträgt.

4. Verfahren gemäß irgendeinem der Ansprüche 1 bis 3, wobei die Nierenzellen in dem Gefrierschritt durch langsames Gefrieren eingefroren werden.

5. Verfahren gemäß irgendeinem der Ansprüche 1 bis 4, wobei die kultivierten Nierenzellen Aggregate, erhalten durch Kultivierung von Nierenzellen in einem Zustand, in dem sie nicht an einem Kulturgefäß haften, umfassen.

6. Verfahren gemäß irgendeinem der Ansprüche 1 bis 5, wobei die Nierenzellen menschliche Primärzellen sind.

7. Verfahren gemäß irgendeinem der Ansprüche 1 bis 6, wobei die Nierenzellen vom proximalen Tubulus abstammende Zellen sind.

## Revendications

1. Procédé de production d'une culture de cellules rénales comprenant :
une étape de récupération destinée à récupérer des cellules rénales mises en culture ; et
une étape de congélation destinée à congeler les cellules rénales récupérées à l'étape de récupération, et
une étape de mise en culture destinée à décongeler des cellules rénales à l'état congelé,
dans lequel :
les cellules rénales sont récupérées sous forme d'agrégat à l'étape de récupération, et
l'agrégat est congelé tandis qu'un état agrégé de l'agrégat est maintenu à l'étape de congélation, et
les cellules rénales sont mises en culture dans un état de non-adhérence sur un récipient de culture pendant 5 jours ou plus à l'étape de mise en culture.

2. Procédé selon la revendication 1, comprenant en outre une étape destinée à ajouter un milieu de cryoconservation aux cellules rénales récupérées entre l'étape de récupération et l'étape de congélation.

3. Procédé selon la revendication 1 ou la revendication 2, dans lequel un nombre de cellules rénales constituant l'agrégat est supérieur ou égal à 100 et inférieur ou égal à 5 000.

4. Procédé selon l'une quelconque des revendications 1 à 3, dans lequel les cellules rénales sont congelées par congélation lente à l'étape de congélation.

5. Procédé selon l'une quelconque des revendications 1 à 4, dans lequel les cellules rénales mises en culture incluent des agrégats obtenus par mise en culture de cellules rénales dans un état de non-adhérence sur un récipient de culture.

6. Procédé selon l'une quelconque des revendications 1 à 5, dans lequel les cellules rénales sont des cellules primaires humaines.

7. Procédé selon l'une quelconque des revendications 1 à 6, dans lequel les cellules rénales sont des cellules dérivées de tubule proximal.
